# EUROPEAN PATENT SPECIFICATION

(11) **EP 4 385 424 B1**
(45) Date of publication and mention of the grant of the patent: **06.08.2025**
(21) Application number: 23209491.2
(22) Date of filing: 13.11.2023
(51) Int. Cl.: A61B 10/00, A01K 29/00, A61D 17/00

(54) **INVASIVE DEVICE FOR MEASURING THE TEMPERATURE EVOLUTION**
INVASIVE VORRICHTUNG ZUR MESSUNG DER TEMPERATURENTWICKLUNG
DISPOSITIF INVASIF POUR MESURER L'ÉVOLUTION DE LA TEMPÉRATURE

(30) Priority: 12.12.2022 BE 202206007
(43) Date of publication of application: 19.06.2024
(73) Proprietor: Van Hyfte, Luc, 9052 Zwijinaarde (BE)
(72) Inventor: Van Hyfte, Luc, 9052 Zwijinaarde (BE)
(74) Representative: Bureau M.F.J. Bockstael NV

(56) References cited:
- BE-A7- 1 015 962
- JP-A- 2004 180 828
- US-A1- 2009 312 667

## Description

The present invention relates to invasive intra-vaginal devices for measuring a temperature progression in the vagina of mammals, more specifically cattle.

In particular, the invention is intended for predicting an impending delivery in cattle.

Probably already since people first started breeding cattle, there has been a desire to predict impending deliveries of pregnant animals, more specifically cattle. Indeed, a significant portion of the deliveries does not go entirely as planned. It is thus important out of financial considerations of the cattle breeder as well as from an animal welfare perspective to follow up deliveries. Adequately following up the delivery and intervening in time in case of complications, if relevant by calling in expert help, can indeed reduce negative effects, such as for example possible damage of the birth canal, or may mean the difference between life and death of both the calf (or calves) and the pregnant or calving cow. For monitoring purposes it is of course important first and foremost to predict as well as possible when the delivery will take place.

It is known that approximately 24 hours before the delivery the temperature in the vagina of the cow drops. Said drop is typically between approximately 0.5 and 1°C. Traditionally, the temperature was monitored by taking manual measurements whereby a thermometer was periodically inserted into the vagina of the cow. However, this approach was rather prone to errors, among others because in practice it is difficult to always take the measurement in the same place in the vagina. Furthermore, periodically taking the measurement was taxing and not comfortable for the cow as well as being very impractical and inefficient for the cattle breeder. Indeed, the latter always had to go to the particular animal at fixed times and interrupt their activities or night's rest.

Furthermore, it is known that modern cattle breeders use probes with temperature sensors that are vaginally inserted into a cow for longer periods of time from 2 to 3 weeks before the scheduled calving and transmit periodic temperature values. However, known disadvantages are that said probes can be very uncomfortable for the cow and give rise to vaginal irritations and infection. Furthermore, it is difficult to stabilise the probes in the vagina such that the measurements are always taken in approximately the same location without causing internal damage.

JP2004180828 discloses a probe with a sensor housed in a capsule mounted in the probe. The probe contains a hollow section that can expand or shrink, whereby said hollow section in non-expanded form is elongated and in expanded form assumes a balloon-like form. When in use, the probe, in balloon-like form, is pressed against the wall of the vagina. When in use, secretions such as urine are discharged via the space between the outer wall of the probe and the wall of the vagina. Because the probe touches the vagina, there are obstacles in said space, in other words there is no free passage which may negatively affect the stability of the probe in case of sustained insertion and furthermore may cause inconvenience and discomfort to the animal.

The purpose of the present invention is to provide an alternative that provides a solution to at least one of the aforementioned and other disadvantages.

To this end, the invention relates to an intra-vaginal device for measuring a temperature progression in the vagina, comprising an inner wall, an elastic outer wall connected to the inner wall, an inflatable space between the inner wall and the outer wall, a sealable connection to the inflatable space through which in open condition a gas can be blown, respectively in or out of the inflatable space, and a temperature sensor. Preferably, the temperature sensor is mounted on or in the outside of the outer wall. Furthermore, it is characteristic that the inner wall of the device delimits a hollow space with an opening in each of its facing ends acting as a passage for discharging secretions.

The device, which typically substantially has the form of a sleeve or tube with open ends, after at least partially being inserted into the vagina, can be inflated, by blowing the gas into the inflatable space, until the outside of the outer wall of the device presses against the inner wall of the vagina such that it is wedged in. The device is then in operational condition such that the measurements can be taken periodically using the temperature sensor. The gas used is typically air but obviously another gas can also be used.

As soon as the device is in operational condition and if relevant after measuring and registering the pressure in the inflatable space the sealable connection can be sealed. If relevant the sealable connection can be opened periodically to measure the pressure in the inflatable chamber and to compensate possible pressure changes by blowing in extra gas, for example. Alternatively, continuous monitoring and, if necessary, adjustment of the pressure in the sealable space, can be opted for.

An advantage of the device according to the invention is that it can be inserted easily thanks to the smaller dimensions in uninflated condition. Furthermore, because the outer wall is elastic, the risk of internal damage is reduced and the animal's comfort is increased.

A further advantage is that, while the device is wedged in the vagina, secretions, such as for example urine, can be easily and effectively discharged without obstacles via the passage delimited by the inner wall centrally in the device. Typically, the passage has the form of a sleeve or tube with open ends. This smooth discharge through the device barely differs from the discharge of secretions of the animal in normal condition, i.e. without inserted device, and contributes to the sustained insertion of the device without or practically without any inconvenience for the animal. Sustained insertion, over the space of days or weeks, is indeed required for predicting an impending delivery.

In a preferred embodiment, the outside of the outer wall of the device is provided with one or several protrusions. Providing said protrusions reduces the risk of the device shifting unintentionally when the device is wedged in the vagina. This increases the reliability of the measurements. The protrusions can be discrete protrusions which are distributed randomly or according to a preset pattern across the outer wall or over a section of the outer wall, such as for example, equidistant from each other. The protrusions can also be continuous such as for example ring- or spiral-shaped. This is possible if the device has the form of a sleeve or tube, for example.

In one embodiment, optionally the device may be inserted only partially into the vagina such that, in operational condition, when the device is inserted at least partially into the vagina, the sealable connection is externally accessible.

In a further embodiment, the sealable connection comprises a valve mounted in the outer wall of the device. Typically, the device has a substantially elongated form. The valve can be mounted in the outer wall close to a first end of a device which, when the device is inserted, is located closest to the body opening such that is it easier to access. In case the device in operational condition is only partially inserted and therefore still has a protruding externally accessible part, the valve is preferably on the external accessible part. This reduces the risk of vaginal infections. Preferably, the temperature sensor is mounted close to a second end of the device, opposite the first end such that in operational condition the second end is oriented toward the opening of the cervix and therefore the measurements can be taken as closely as possible to the opening of the cervix.

In a further embodiment, the inner wall of the device, which typically is shaped like a sleeve or tube, is made from an elastic material. The elasticity of the inner wall and the outer wall can be different or the same. Alternatively, the inner wall of the device can be executed as a rigid body such as a sleeve or a tube made from synthetic material or metal, such as for example steel.

In a further embodiment the temperature sensor comprises a wireless transmitter, for example in the form of a transponder. This allows the periodic measurements to be transmitted via the wireless network and as such to be accessible and processed via the mobile phone or computer of the cattle breeder. Alternatively the temperature sensor can be further provided with a data processing unit such that the measured data can be already wholly or partly processed locally and, for example, a signal is transmitted when the changing temperature values signal the moment of delivery. The temperature sensor can be further provided with a microcontroller which allows the duration and frequency of the measurements to be modified, remotely if relevant.

With the intention of better showing the characteristics of the invention, a few preferred variants of the device according to the invention are described hereinafter by way of an example, without any limiting nature, with reference to the accompanying drawings, wherein:
Figure 1 schematically shows a vagina of a cow;
figure 2 schematically shows the vagina of figure 1 in which a device according to an embodiment of the present invention is partially inserted, whereby the device is in uninflated condition;
figure 3 schematically shows the vagina of figure 1 in which a device according to an embodiment of the present invention is partially inserted, whereby the device is in inflated operational condition;
figure 4 and 5 schematically show a part of a device, each according to an embodiment of the present invention.

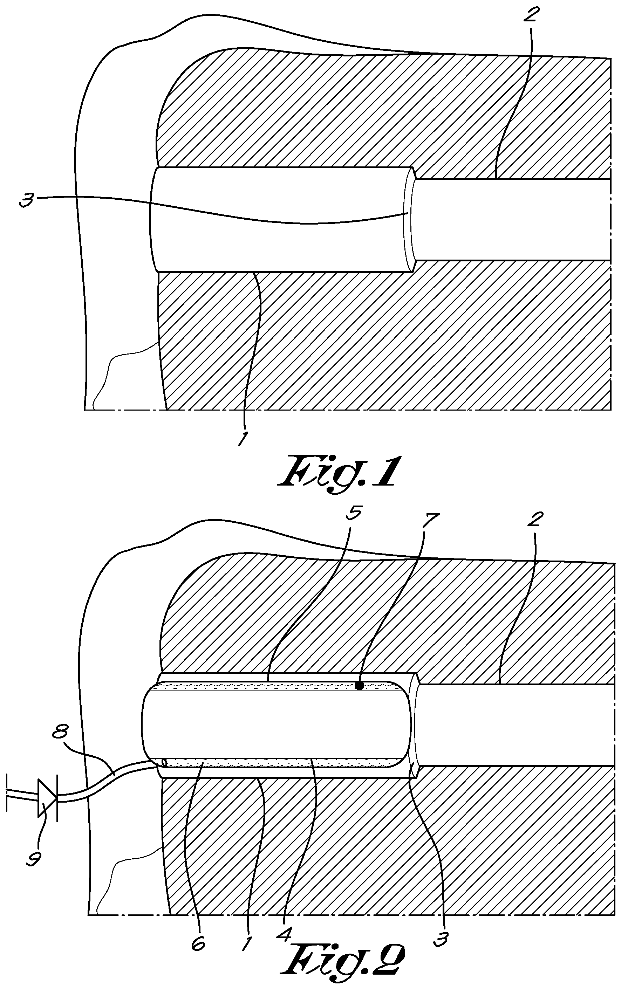

Figure 1 schematically shows the vagina 1 of a cow that connects with the cervix 2 via the opening of the cervix 3.

Figure 2 schematically shows a device for measuring the temperature progression in the vagina whereby the device is partially inserted into the vagina 1. The device comprises an elongated double-walled elastic sleeve with open ends, one end of which extends close to the opening of the cervix 3, and at the opposite end still protrudes out of the body. The outer wall 5 of the sleeve is connected to the inner wall 4 such that one inflatable space 6 is formed between the inner wall 4 and the outer wall 5. The inner wall 4 delimits a hollow space centrally in the sleeve which forms a passage for discharging secretions through the sleeve via the open ends. The inner wall and the outer wall are made from an elastic material such as for example rubber or polyurethane. The inner wall and the outer wall can be made from the same or a different material.

An air hose 8 is connected to the inflatable space 6. At its free end, the air hose is provided with a valve 9. Alternatively, instead of an air hose with valve, the valve can also be directly provided in the outer wall of the device. In both cases there is a sealable connection to the inflatable space 6.

On or in the outside of the outer wall 5, a temperature sensor 7 is provided close to the opening of the cervix 3. Temperature sensors, such as for example contactless infrared temperature sensors, are state of the art and available in different embodiments. Preferably, the temperature sensor is executed as an integrated circuit using techniques as known in the semiconductor industry and the thin film industry whereby the functional parts are enveloped and shielded by a suitable synthetic or epoxy layer, for example against penetrating fluid. The IC temperature sensor can be mounted on a flexible support. The attachment on or in the outer wall of the sleeve can be done by means of gluing, for example. Together with the temperature sensor, batteries and a wireless transmitter are provided. This allows the temperature to be periodically measured and the measured values to be transmitted such that remote monitoring is possible by the cattle breeder via their mobile phone, tablet or computer using a wireless network, for example. The transmitted data can, if relevant, be coupled with the data relating to the identity of the cow present in the neck transponder of the cow.

Other components can also be integrated in the temperature sensor such as a wireless receiver, a transponder, a microcontroller and a data processing unit. Depending on the chosen composition of components this allows the temperature to be periodically measured, if relevant the duration of the measurement and/or the measurement frequency to be modified remotely, the measurement data to be processed completely or partly and if relevant a specific signal to be transmitted if the processing of the measurement data shows a temperature drop to have taken place such that the time of the delivery can be predicted.

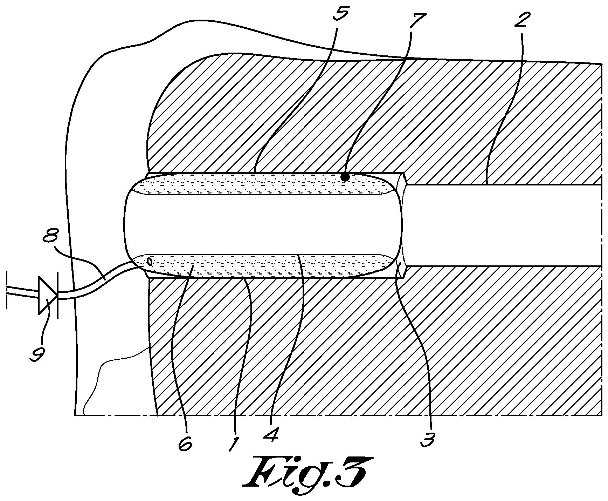

Figure 3 shows figure 2 but then with the device for measuring the temperature progression in the vagina in operational condition after inflating the sleeve by blowing air into the inflatable space 6. The outer wall 5 of the sleeve presses against the vaginal wall in such a way that the sleeve is wedged in by the vagina 1. The valve 9 is closed such that no air can escape from the inflatable space 6 via the air hose 8 and the sleeve stays in place.

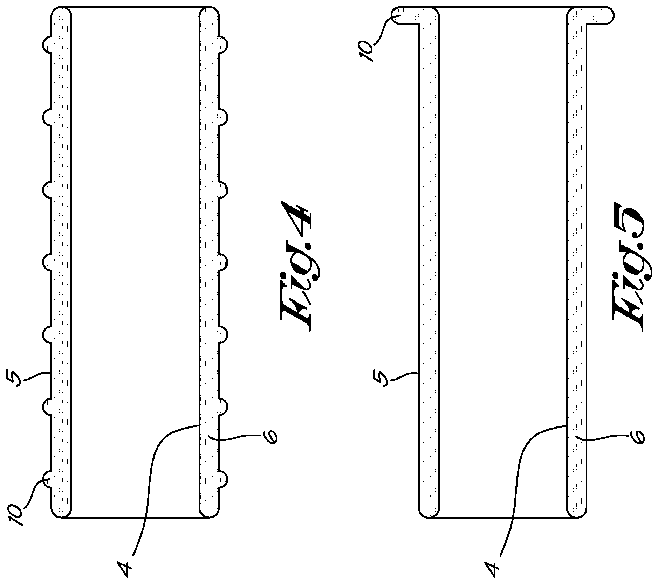

Figures 4 and 5 show a variant of the sleeve of the device according to figure 2, whereby the outer wall 5 of the sleeve is provided with protrusions 10. In figure 4 the protrusions are applied over practically the entire length of the sleeve. In figure 5 the protrusions 10 are only applied close to the end of the sleeve such that in operational condition, the device is located as closely as possible to the opening of the cervix 3. The protrusions 10 of figure 5 can be a single protrusion which extends across the entire contour of a transverse segment of the sleeve or several protrusions applied on the contour of the transverse segment. The elastic protrusions 10 ensure a good and comfortable stability of the device in the vagina.

The application and the operation of the device for measuring the temperature progression in the vagina substantially consists of a number of steps. In a first step, the device in an embodiment as described above or a random combination thereof is at least partially inserted into the vagina 1 of the cow in uninflated condition, whereby the section provided with the sealable connection 8 to the inflatable space 6 remains external or close to the body opening and whereby preferably the other end of the device is located close to the opening of the cervix 3 such that the temperature sensor 7 is also located close to the opening of the cervix 3. As the device is not in an inflated condition this can be done relatively easily due to the limited diameter.

The device can be put in operational condition by blowing air into the inflatable space 6 via the sealable connection 8. This can be done, for example, by opening the valve 9 and connecting the sealable connection to a pump in an airtight way, and subsequently, if relevant using pressure control, blowing in a quantity of air to such an extent that the outer wall 5 of the device touches the inner wall of the vagina and thus is wedged in. The connection to the pump can then be broken and the valve 9 closed.

When the device is in operational condition, the periodic measurements can be started and the measured values of the temperature sensor 7 can be remotely communicated via a contactless transmitter.

Optionally, the measurement data can also be processed by a data processing unit, integrated together with the temperature sensor 7 or as a component of the receiver, such as for example the mobile phone or computer of the cattle breeder. If the processing of the measurement data shows a temperature drop has taken place such that the time of the delivery can be predicted, a warning signal can be given.

To remove the device from the vagina, first the sealable connection 8 is opened such that air is blown out of the inflatable space 6. As it is no longer wedged in, the device can subsequently be simply removed.

The present invention is by no means limited to the embodiment(s) described as an example and shown in the figures, but a device according to the invention can be realised in all kinds of forms and dimensions without departing from the scope of the invention, as defined by the appended claims.

## Claims

1. An intra-vaginal device for measuring a temperature progression in the vagina (1), comprising:
an inner wall (4),
an elastic outer wall (5) connected to the inner wall,
an inflatable space (6) between the inner wall and the outer wall,
a sealable connection (8) to the inflatable space through which in open condition a gas can be blown into or out of, respectively, the inflatable space, whereby the device, in operational condition, when at least partially inserted into the vagina by blowing the gas into the inflatable space can be inflated until the outside of the outer wall of the device presses against the inner wall of the vagina such that it is wedged in, and
a temperature sensor (7), **characterised in that** the inner wall (4) of the device delimits a hollow space with an opening in each of its facing ends acting as a passage for discharging secretions.

2. The device according to claim 1, **characterised in that** the outside of the outer wall (5) is provided with one or several protrusions (10).

3. The device according to any one of the previous claims, **characterised in that**, in operational condition, when the device is at least partially inserted into the vagina, the sealable connection (8) is externally accessible.

4. The device according to any one of the previous claims, **characterised in that** the temperature sensor (7) is mounted on or in the outside of the outer wall (5).

5. The device according to any one of the previous claims, **characterised in that** the sealable connection (8) comprises a valve (9) mounted in the outer wall (5).

6. The device according to claim 5, **characterised in that** the device has a substantially elongated form and the valve (9) is mounted in the outer wall (5) close to a first end.

7. The device according to claim 6, **characterised in that** the temperature sensor (7) is mounted close to a second end of the device, opposite the first end.

8. The device according to any one of the previous claims, **characterised in that** the inner wall (4) consists of elastic material.

9. The device according to any one of the claims 1 to 7, **characterised in that** the inner wall (4) is executed as a rigid body.

10. The device according to any one of the previous claims, **characterised in that** the temperature sensor (7) comprises a wireless transmitter.

11. The device according to any one of the previous claims, **characterised in that** the temperature sensor (7) comprises a microcontroller.

12. The device according to any one of the previous claims, **characterised in that** the temperature sensor (7) comprises a data processing unit.

13. The device according to claim 10 or according to claims 11, 12, when depending on claim 10, **characterised in that** the wireless transmitter is a transponder.

## Patentansprüche

1. Intravaginale Vorrichtung, die dazu bestimmt ist, einen Temperaturverlauf in der Vagina (1) zu messen, die Folgendes umfasst:
eine innere Wand (4);
eine äußere elastische Wand (5), die mit der inneren Wand verbunden ist;
einen aufblasbaren Raum (6) zwischen der inneren Wand und der äußeren Wand;
eine versiegelbare Verbindung (8) zum aufblasbaren Raum, über die im geöffneten Zustand ein Gas in den aufblasbaren Raum eingeblasen bzw. aus diesem abgesaugt werden kann; wobei die Vorrichtung im Betriebszustand, wenn sie zumindest teilweise in die Vagina eingeführt ist, durch Einblasen des Gases in den aufblasbaren Raum so weit aufgeblasen werden kann, dass die äußere Seite der äußeren Wand der Vorrichtung derart gegen die innere Wand der Vagina drückt, dass diese in diese eingedrückt wird; und
einen Temperatursensor (7);
**dadurch gekennzeichnet, dass** die innere Wand (4) der Vorrichtung einen Hohlraum begrenzt, der an jedem seiner gegenüberliegenden Enden eine Öffnung aufweist, die als Durchgang für die Ableitung von Sekreten dient.

2. Vorrichtung nach Anspruch 1, **dadurch gekennzeichnet, dass** die äußere Seite der äußeren Wand (5) mit einem oder mehreren Vorsprüngen (10) versehen ist.

3. Vorrichtung nach einem der vorstehenden Ansprüche, **dadurch gekennzeichnet, dass** im Betriebszustand, wenn die Vorrichtung zumindest teilweise in die Vagina eingeführt ist, die versiegelbare Verbindung (8) von außen zugänglich ist.

4. Vorrichtung nach einem der vorstehenden Ansprüche, **dadurch gekennzeichnet, dass** der Temperatursensor (7) an der äußeren Seite der äußeren Wand (5) oder in der betreffenden Seite angebracht ist.

5. Vorrichtung nach einem der vorstehenden Ansprüche, **dadurch gekennzeichnet, dass** die versiegelbare Verbindung (8) ein Ventil (9) umfasst, das in der äußeren Wand (5) angebracht ist.

6. Vorrichtung nach Anspruch 5, **dadurch gekennzeichnet, dass** die Vorrichtung eine im Wesentlichen längliche Form aufweist und das Ventil (9) in der äußeren Wand (5) in der Nähe eines ersten Endes angebracht ist.

7. Vorrichtung nach Anspruch 6, **dadurch gekennzeichnet, dass** der Temperatursensor (7) in der Nähe eines zweiten Endes der Vorrichtung angebracht ist, das dem ersten Ende gegenüberliegt.

8. Vorrichtung nach einem der vorstehenden Ansprüche, **dadurch gekennzeichnet, dass** die innere Wand (4) aus einem elastischen Material besteht.

9. Vorrichtung nach einem der Ansprüche 1 bis 7, **dadurch gekennzeichnet, dass** die innere Wand (4) als starre Wand ausgebildet ist.

10. Vorrichtung nach einem der vorstehenden Ansprüche, **dadurch gekennzeichnet, dass** der Temperatursensor (7) einen drahtlosen Sender umfasst.

11. Vorrichtung nach einem der vorstehenden Ansprüche, **dadurch gekennzeichnet, dass** der Temperatursensor (7) einen Mikrocontroller umfasst.

12. Vorrichtung nach einem der vorstehenden Ansprüche, **dadurch gekennzeichnet, dass** der Temperatursensor (7) eine Datenverarbeitungseinheit umfasst.

13. Vorrichtung nach Anspruch 10 oder nach den Ansprüchen 11, 12, wenn sie von Anspruch 10 abhängen, **dadurch gekennzeichnet, dass** der drahtlose Sender ein Transponder ist.

## Revendications

1. Dispositif intravaginal qui est destiné à mesurer une progression de la température dans le vagin (1), qui comprend :
une paroi interne (4) ;
une paroi élastique externe (5) qui est reliée à la paroi interne ;
un espace gonflable (6) entre la paroi interne et la paroi externe ;
un raccordement scellable (8) à l'espace gonflable, par l'intermédiaire duquel, à l'état ouvert, un gaz peut être insufflé dans l'espace gonflable ou évacué de ce dernier, respectivement ; dans lequel le dispositif, en état de mise en service, lorsqu'il est inséré au moins en partie dans le vagin en insufflant le gaz dans l'espace gonflable, peut être gonflé jusqu'à ce que le côté extérieur de la paroi externe du dispositif exerce une pression contre la paroi interne du vagin d'une manière telle qu'il s'y retrouve enfoncé ; et
un capteur de la température (7) ; **caractérisé en ce que**
la paroi interne (4) du dispositif délimite un espace creux qui comporte une ouverture dans chacune de ses extrémités opposées, qui fait office de passage pour l'évacuation de sécrétions.

2. Dispositif selon la revendication 1, **caractérisé en ce que** le côté extérieur de la paroi externe (5) est muni d'une ou de plusieurs protrusions (10).

3. Dispositif selon l'une quelconque des revendications précédentes, **caractérisé en ce que**, en état de mise en service, lorsque le dispositif a été inséré au moins en partie dans le vagin, le raccordement scellable (8) est accessible depuis l'extérieur.

4. Dispositif selon l'une quelconque des revendications précédentes, **caractérisé en ce que** le capteur de la température (7) est monté sur le côté extérieur de la paroi externe (5) ou dans le côté en question.

5. Dispositif selon l'une quelconque des revendications précédentes, **caractérisé en ce que** le raccordement scellable (8) comprend une valve (9) qui est montée dans la paroi externe (5).

6. Dispositif selon la revendication 5, **caractérisé en ce que** le dispositif possède une forme de configuration essentiellement allongée et la valve (9) est montée dans la paroi externe (5) à proximité d'une première extrémité.

7. Dispositif selon la revendication 6, **caractérisé en ce que** le capteur de la température (7) est monté à proximité d'une deuxième extrémité du dispositif, qui est opposée à la première extrémité.

8. Dispositif selon l'une quelconque des revendications précédentes, **caractérisé en ce que** la paroi interne (4) est constituée d'une matière élastique.

9. Dispositif selon l'une quelconque des revendications 1 à 7, **caractérisé en ce que** la paroi interne (4) est réalisée à la manière d'une paroi rigide.

10. Dispositif selon l'une quelconque des revendications précédentes, **caractérisé en ce que** le capteur de la température (7) comprend un émetteur sans fil.

11. Dispositif selon l'une quelconque des revendications précédentes, **caractérisé en ce que** le capteur de la température (7) comprend un m`icroprocesseur.

12. Dispositif selon l'une quelconque des revendications précédentes, **caractérisé en ce que** le capteur de la température (7) comprend une unité de traitement de données.

13. Dispositif selon la revendication 10 ou selon les revendications 11, 12, lorsqu'elles dépendent de la revendication 10, **caractérisé en ce que** l'émetteur sans fil est un transpondeur.
